# EUROPÄISCHE PATENTSCHRIFT

(11) **EP 0 770 605 B1**
(45) Veröffentlichungstag und Bekanntmachung des Hinweises auf die Patenterteilung: **10.01.2001**
(21) Anmeldenummer: 96810697.1
(22) Anmeldetag: 16.10.1996
(51) Int. Cl.: C07D 303/16, C09D 133/06, C08G 59/24

(54) **Pulverlack**
Powder paint
Peinture en poudre

(30) Priorität: 25.10.1995 CH 301495
(43) Veröffentlichungstag der Anmeldung: 02.05.1997
(73) Patentinhaber: Vantico AG, 4057 Basel (CH)
(72) Erfinder: Gottis, Philippe-Guilhaume, 68200 Mulhouse (FR)

(56) Entgegenhaltungen:
- EP-A- 0 536 085
- DE-A- 2 654 306
- US-A- 3 859 314
- CHEMICAL ABSTRACTS, vol. 72, no. 20, 18.Mai 1970 Columbus, Ohio, US; abstract no. 101295, BUSSO, C.J., E.A.: "Chemical structure-mechanical properties studies on pure epoxy resin systems" XP002025589 & PROC., ANNIV. CONF., SPI, (SOC. PLAST. IND.), REINF. PLAST./COMPOS. DIV., 25TH (1970), 3-B.,

## Beschreibung

Die vorliegende Erfindung betrifft einen Pulverlack auf Basis eines oder mehrerer Poly(meth)acrylharze mit freien Carboxylgruppen, enthaltend einen Härter auf Basis von Epoxidverbindungen mit einem Molekulargewicht von höchstens 1500 sowie die Verwendung dieses Pulverlacks als Automobildecklack.

Epoxidverbindungen mit einem Molekulargewicht von höchstens 1500 und ihre Verwendung zur Herstellung härtbarer Zusammensetzungen sind bereits vielfach beschrieben worden.

Beispielsweise offenbart die DE-A-26 54 306 die Verwendung von entsprechenden Glycidylestern, unter anderem von trans-Hexahydroterephthalsäurediglycidylester, für die Umhüllung elektronischer Bauteile und Chemical Abstracts, Vol. 72, No. 101295 härtbare Gemische bestehend aus verschiedenen Epoxidharzen, unter anderem auch aus trans-Hexahydroterephthalsäurediglycidylester, und m-Dianilin sowie die mechanischen Eigenschaften des hieraus erhältlichen, gehärteten Materials.

Härter für Pulverlacke auf Basis von Epoxidverbindungen mit einem Molekulargewicht von höchstens 1500 sind ebenfalls bekannt und werden insbesondere dann eingesetzt, wenn das Bindemittel des Pulverlacks freie Carboxylgruppen aufweist.

Beispielsweise beschreibt die EP-A-0 536 085 die Verwendung von Hexahydrophthalsäurediglycidylester und Hexahydroterephthalsäurediglycidylester zusammen mit weiteren flüssigen Epoxidharzen in Form einer festen Lösung dieser Komponenten als Härter für Pulverlacke auf Basis von Polyestern mit freien Carboxylgruppen. Hexahydroterephthalsäurediglycidylester ist hierbei nur in genereller Weise offenbart, d. h. ohne einen Unterschied zwischen cis- und trans-Form zu machen.

Weiterhin ist beispielsweise aus Hohnson Wax Speciality Chemicals Product Application Bulletin, Powder Coatings ein Pulverlack bekannt, der Triglycidylisocyanurat als Härter und ein Poly(meth)acrylharz mit freien Carboxylgruppen als Bindemittel enthält. Diese Pulverlacke haben jedoch unter anderem den Nachteil, dass sie nur bei relativ hohen Weiterhin ist beispielsweise aus Hohnson Wax Speciality Chemicals Product Application Bulletin, Powder Coatings ein Pulverlack bekannt, der Triglycidylisocyanurat als Härter und ein Poly(meth)acrylharz mit freien Carboxylgruppen als Bindemittel enthält. Diese Pulverlacke haben jedoch unter anderem den Nachteil, dass sie nur bei relativ hohen Temperaturen einen ausreichend guten Verlauf zeigen und daher für die Anwendung bei Temperaturen von 140 bis 150°C nicht geeignet sind, wie es z.B. für Automobildecklacke gefordert wird.

Es wurde nunmehr gefunden, dass Pulverlacke auf Basis eines oder mehrerer Poly(meth)acrylharze mit freien Carboxylgruppen, die einen bei Raumtemperatur (15° bis 30°C) festen Härter auf Basis niedrigmolekularer Epoxidverbindungen enthalten, dessen wesentlicher Bestandteil 1,4-Cyclohexandicarbonsäurediglycidylester in Form des reinen trans-Isomeren oder eines cis-/trans-lsomerengemisches mit einem Schmelzpunkt oberhalb von 70°C ist, den gewünschten guten Verlauf zeigen, auch wenn sie bei Temperaturen im Bereich von 130 bis 150°C verarbeitet, d.h. appliziert und eingebrannt werden.

Die vorliegende Erfindung betrifft daher einen Pulverlack auf Basis eines oder mehrerer Poly(meth)acrylharze mit freien Carboxylgruppen, enthaltend einen Härter auf Basis von Epoxidverbindungen mit einem Molekulargewicht von höchstens 1500, der bei Raumtemperatur fest ist, wobei die Epoxidverbindungen aus 60 bis 100 Gewichtsprozent 1,4-Cyclohexandicarbonsäurediclycidylester in Form des reinen trans-Isomeren oder eines cis/trans-lsomerengemisches mit einem Schmelzpunkt (definiert als Peakmaximum im DSC-Scan bei einer Aufheizgeschwindigkeit von 10°C pro Minute) oberhalb von 70°C, und 0 bis 40 Gewichtsprozent weiteren Epoxidverbindungen bestehen, wobei 0 bis 15 Gewichtsprozent der gesamten Epoxidverbindungen aromatische Strukturelemente im Molekül aufweisen können.

Weitere Gegenstände der Erfindung bilden Pulverlacke auf Basis eines oder mehrerer Poly(meth)acrylharze, die den erfindungsgemässen Pulverlackhärter enthalten, sowie deren Verwendung als Automobildecklack.

Neben dem gewünschten Verlaufsverhalten zeigen erfindungsgemässe Pulverlacke bei guter Reaktivität im Temperaturbereich von 130°C bis 150°C eine sehr gute Lagerstabilität bei Raumtemperatur bis zu mässig erhöhten Temperaturen, im Bereich unter 40°C beispielsweise, zeichnen sich also durch ein gutes Reaktivitäts-/Stabilitätsverhältnis aus. Die erfindungsgemäss als Pulverlackhärter vorgesehene Form von 1,4-Cyclohexandicarbonsäurediglycidylester wirkt zudem kaum plastifizierend und zeigt keinerlei Neigung zum Verklumpen, was die Verarbeitung der Zusammensetzungen zu Pulverlacken, namentlich das Zerkleinern der zuvor beispielsweise im Extruder homogenisierten Masse wesentlich erleichtert, und das Verbacken während der Lagerung verhindert.

Erfindungsgemäss als Härter einsetzbare Formen von 1,4-Cyclohexandicarbonsäurediclycidylester sind in einfacher Weise z.B. dadurch erhältlich, dass man Isomerengemisch von trans- und cis-1,4-Cyclohexandicarbonsäure, wie es auch kommerziell verfügbar ist, mit Wasser einer Temperatur von etwa 80 bis 95°C behandelt, wobei sich das cis-lsomere löst, so dass eine in ausreichendem Ausmass mit dem trans-Isomer angereicherte Form der 1,4-Cyclohexandicarbonsäure erhalten wird. Diese kann dann in üblicher Weise durch Umsetzung mit Epichlorhydrin glycidylisiert werden, wie z.B. im U.S.-Patent (US-A-)3,859,314 beschrieben ist.

Obwohl erfindungsgemässe Pulverlacke bevorzugt sind, bei denen die Epoxidverbindungen zu 100 Gewichtsprozent aus 1,4-Cyclohexandicarbonsäurediclycidylester in Form eines reinen trans-Isomeren oder eines cis-/trans-lsomerengemisches mit einem Schmelzpunkt oberhalb von 70°C bestehen, können die Epoxidverbindungen des Härters auch aus bis zu 30 bis 40 Gewichtsprozent anderen Epoxidverbindungen, bevorzugt anderen Di- oder Polyglycidylestern, bestehen.

Bis zu 15 Gewichtsprozent der gesamten Epoxidverbindungen eines erfindungsgemässen Pulverlacks können hierbei aromatische Strukturelemente enthalten, ein höherer Anteil ist wegen der dann stark absinkenden Wetterbeständigkeit nicht empfehlenswert. Diese Epoxidverbindungen umfassen beispielsweise die entsprechenden Glycidylester, die in der EP-A-0 506 617 und in der EP-A-0 536 085, deren beider Beschreibung als Bestandteil dieser Beschreibung betrachtet wird, als Härter für Pulverlacke auf Basis saurer Polyester genannt sind und die aromatische Gruppen enthalten, insbesondere um die Diglycidylester von Phthal-, Isophthal- oder Terephthalsäure oder die Triglycidylester von Trimesin- oder Trimellitsäure.

Grössere Mengen anderer Epoxidverbindungen als 15 Gewichtsprozent oder ein darüberhinausgehender zusätzlicher Anteil weiterer Epoxidverbindungen ist möglich, wenn es sich um aliphatische oder cycloaliphatische Epoxidverbindungen handelt.

Bei den aliphatischen Epoxidverbindungen handelt es sich zweckmässigerweise um Polyglycidylester aliphatischer Di- oder Polycarbonsäuren, bevorzugt um die Polyglycidylester von aliphatischen Polycarbonsäuren mit 2 bis 50, besonders bevorzugt mit 2 bis 20 Kohlenstoffatomen, die gegebenenfalls neben den Carboxylgruppen auch andere funktionelle Gruppen aufweisen können. Beispiele für geeignete aliphatische Polycarbonsäuren sind Oxalsäure, Malonsäure, Bernsteinsäure, Glutarsäure, Adipinsäure, Pimelinsäure, Korksäure, Azelainsäure oder Sebacinsäure. Auch aliphatische Polyglycidylester der nachfolgenden Formel (I), worin X₁, X₂, X₃, X₄, X₅ und X₆ unabhängig voneinander ein Wasserstoffatom, eine C₁-C₄-Alkylgruppe oder eine Gruppe der nachstehenden Formel (II) bedeuten und A in Formel (II) für eine Alkylengruppe mit 2 bis 4 Kohlenstoffatome, bevorzugt für eine Ethylengruppe, und Y für ein Wasserstoffatom oder eine Methylgruppe stehen, sind als Härter für die Zwecke der vorliegenden Erfindung geeignet: 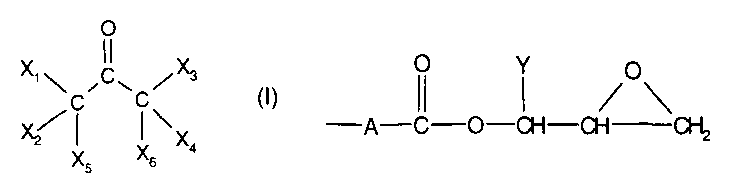 Polyglycidylester der Formel (I) sind z.B. in der EP-A-0 506 617 beschrieben. Spezielle Beispiele sind Aceton-1,1,3,3-tetraproprionsäureglycidylester und Pentanon-(3)-2,2,4,4-tetrapropionsäureglycidylester.

Bevorzugte Polyglycidylester aliphatischer Polycarbonsäuren sind die Diglycidylester von Oxalsäure, Bernsteinsäure, Adipinsäure, Sebacinsäure, Azelainsäure.

Bei den cycloaliphatischen Epoxidverbindungen handelt es sich zweckmässigerweise um Polyglycidylester cycloaliphatischer Di- oder Polycarbonsäuren d.h. um Polyglycidylester von Di- und Polycarbonsäuren auf Basis eines Kohlenstoffgerüstes, das einen oder mehrere cycloaliphatische Ringe enthält und frei von C-C-Mehrfachbindungen oder von aromatischen Gruppen ist. Die einzelnen cycloaliphatischen Ringe können einen oder mehrere Substituenten, z.B. C₁-C₆-Alkyl-, Chlor-, Brom- oder Hydroxylsubstituenten aufweisen und haben bevorzugt 5 bis 10 Kohlenstoffatome. Enthält die Polycarbonsäure zwei oder mehrere cycloaliphatische Ringe, so können diese annelliert oder über geeignete Atomgruppen verknüpft sein, insbesondere über geradkettige oder verzweigte 2 bis 6-wertige aliphatische Gruppen von beispielsweise 1 bis 30 Kohlenstoffatomen, die auch eines oder mehrere Heteroatome, wie z.B. Schwefel-, Stickstoff- oder insbesonders Sauerstoffatome enthalten sowie Substituenten, beispielsweise Chlor-, Brom- oder Hydroxylsubstituenten, aufweisen können. Beispiele für solche Brückengruppen sind die Gruppen der Formel -CH₂-, C(CH₃)₂-, >CH-, >C(CH₃)-, >C< oder 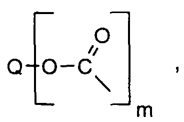 worin Q einen 2 bis 6-wertigen organischen Rest mit bevorzugt 2 bis 15 Kohlenstoffatomen bedeutet, der frei von C-C-Doppelbindungen ist. Spezielle Beispiele für erfindungsgemäss geeignete Polyglycidylester cycloaliphatischer Polycarbonsäuren sind die Diglycidylester von Hexahydrophthalsäure, Hexahydroisophthalsäure, Hexahydroterephthalsäure, Methylhexahydrophthalsäure, 2,5-Dimethylhexahydrophthalsäure, Endomethylenhexahydrophthalsäure, die Diglycidylester der 1,8-, der 2,3- sowie der 2,6-Decalindicarbonsäuren, Cyclohexatricarbonsäuretriglycidylester, wie Hexahydrohemimellitsäuretriglycidylester oder insbesondere Hexahydrotrimesinsäuretriglycidylester sowie Hexahydrotrimellitsäuretriglycidylester, die Tetraglycidylester von Hexahydromellophansäure (Cyclohexan-1,2,3,4-tetracarbonsäure) oder von Hexahydropyrromellitsäure (Cyclohexan-1,2,4,5-tetracarbonsäure). Polyglycidylester von Cyclohexanpolycarbonsäuren können z.B. in üblicher Weise aus den entsprechenden Cyclohexancarbonsäuren durch Umsetzung mit Epichlorhydrin erhalten werden. Die Cyclohexanpolycarbonsäuren können, soweit sie nicht kommerziell verfügbar sind, z.B. durch Hydrierung der entsprechenden Benzolpolycarbonsäuren gemäss der US-A-3,444,237 hergestellt werden.

Für die erfindungsgemässen Pulverlacke sind ebenfalls die cycloaliphatischen Polyglycidylester gut geeignet, die in der DE-A-23 19 815 und EP-A-0 506 617 beschrieben sind. Diese weisen die nachstehende Formel (III) auf, worin n eine ganze Zahl von 2 bis 9, bevorzugt von 2 bis 3, und X₁, X₂, X₃ und X₄ unabhängig voneinander ein Wasserstoffatom, eine C₁-C₄-Alkylgruppe oder eine Gruppe der oben schon genannten Formel (II) bedeuten:

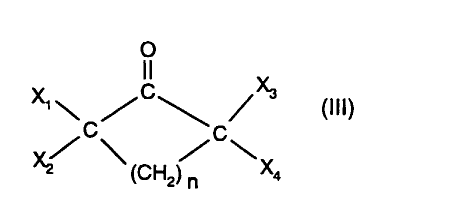

Beispiele für Verbindungen dieses Typs sind Cyclopentanon-2,2,5,5-tetra(propionsäureglycidylester), Cyclohexanon-2,2,6,6-tetra(propionsäureglycidylester) oder Cyclooctanon-2,2-di(propionsäureglycidylester).

Weitere für die vorliegende Erfindung geeignete cycloaliphatische Polyglycidylester sind die ebenfalls in der EP-A-0 506 607 beschriebenen Glycidylester der untenstehenden Formel (IV), worin X₇ einen m-wertigen aliphatischen oder cycloaliphatischen mit 2 bis 30 Kohlenstoffatomen, dessen Kohlenstoffkette gegebenenfalls durch eines oder mehrere Heteroatome, wie Stickstoff- oder Schwefel-, insbesondere aber Sauerstoffatome unterbrochen sein kann und der gegebenenfalls auch substituiert sein, insbesondere Hydroxyl-, Chlor- oder Bromsubstituenten aufweisen kann, m eine ganze Zahl von 2 bis 6 und die Reste X₈ sowie X₉ unabhängig voneinander ein Wasserstoff-, Chlor- oder Bromatom, eine C₁-C₄-Alkylgruppe bedeuten oder aber einer der Reste X₈ oder X₉ eine Gruppe der nachstehenden Formel (V) bedeutet und der andere eine der zuvor angegebenen Bedeutung hat:

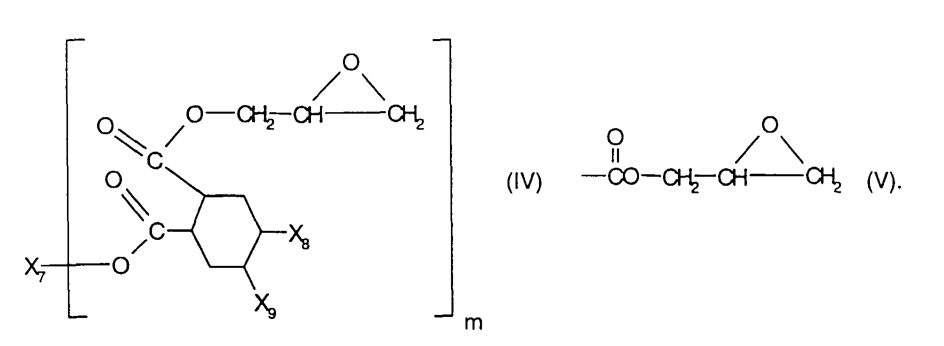

X₇ stellt bevorzugt einen zwei- bis vierwertigen Rest dar, der sich von einem aliphatischen Polyalkohol mit 2 bis 10 Kohlenstoffatomen, der gegebenenfalls oligomerisiert sein kann wie z.B. von Glycol, Glycerin, Trimethylolpropan oder Bis-(trimethylolpropan), oder von Polyetherpolyolen durch Entfernen von zwei bis vier Hydroxylgruppen ableitet oder aber eine zwei- bis vierwertige, bevorzugt zweiwertige Gruppe mit der folgenden Molekülstruktur 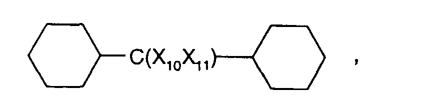 wobei X₁₀ und X₁₁ unabhängig voneinander ein Wasserstoffatom, eine C₁-C₄-Alkyl- oder eine Cyclohexylgruppe darstellen. Besonders bevorzugt stellen X₁₀ und X₁₁ entweder ein Wasserstoffatom oder eine Methylgruppe dar. Speziell bevorzugte Verbindungen dieser Struktur haben die Formeln (G-CH₂)₃C-C₂H₅, (G-CH₂)₂C(C₂H₅)-CH₂-O-CH₂-(C₂H₅)C(CH₂-G)₂, (G-CH₂)₂ CH(OH) sowie G-(C₆H₄)-C(CH₃)₂-(C₆H₄)-G, worin G jeweils einer Gruppe der Formel (VI) entspricht und X₈ entweder ein Wasserstoffatom oder eine Gruppe der oben schon angeführten Formel (V) bedeutet:

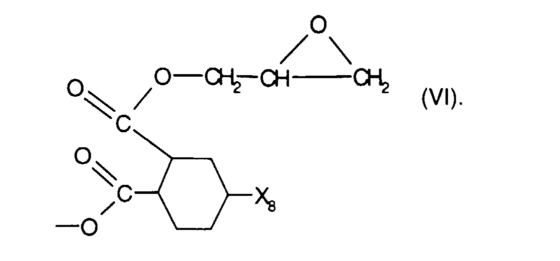

Bevorzugt werden 1,2,4-Cyclohexantricarbonsäuretriglycidylester, 1,3,5-Cyclohexantricarbonsäuretriglycidylester oder Polyglycidylverbindungen der oben schon angeführten Formeln (III) oder (IV) als weitere cyclialiphatische Epoxidverbindungen eingesetzt.

Bei den Epoxidverbindungen, die neben dem 1,4-Cyclohexandicarbonsäurediglycidylester, wahrscheinlich auf Grund ihres geringen Gehaltes an freiem cis-1,4-Cyclohexandicarbonsäurediglycidylester, in den erfindungsgemässen Pulverlackhärtern enthalten sein können, kann es sich um Epoxidharze handeln, die bei Raumtemperatur fest oder flüssig sind. Bei Raumtemperatur flüssige Epoxidverbindungen werden in Form fester Mischphasen (fester Lösungen) eingesetzt, die aus den flüssigen und aus den bei Raumtemperatur festen Epoxidharzen, inclusive der erfindungsgemäss eingesetzten Form des 1,4-Cyclohexandicarbonsäurediglycidylester gebildet sind, wie dies näher in der EP-A-0 536 085 beschrieben ist. Höhere Prozentgehalte an weiteren flüssigen Epoxidverbindungen setzen allerdings den Einsatz einer Form von 1,4-Cyclohexandicarbonsäurediglycidylester voraus, die relativ arm an cis-lsomer ist, da andernfalls keine bei Raumtemperatur festen Zusammensetzungen von Epoxidverbindungen mehr erhältlich sind.

Einen weiteren Gegenstand der Erfindung bildet daher ein Pulverlack, wie oben beschrieben, bei dem die Epoxidverbindungen eine bei Raumtemperatur feste Zusammensetzung enthaltend bei Raumtemperatur feste und bei Raumtemperatur flüssige Epoxidverbindungen bilden, wobei zumindest ein Teil der bei Raumtemperatur festen Epoxidverbindungen in Form einer festen Mischphase oder eines Gemisches mehrerer fester Mischphasen vorliegt und die gesamten bei Raumtemperatur flüssigen Epoxidverbindungen Komponenten einer oder mehrerer der genannten festen Mischphasen bilden.

Beispielsweise kann die genannte feste Zusammensetzung die Epoxidverbindungen in folgendem Verhältnis enthalten:

Beispielsweise kann die genannte feste Zusammensetzung die Epoxidverbindungen in folgendem Verhältnis enthalten:
- 70 bis 95 Gew.-%: 1,4-Cyclohexandicarbonsäurediglycidylester in Form des reinen trans-lsomeren oder eines cis-/trans-lsomerengemisches mit einem Schmelzpunkt oberhalb von 70°C,
- 5 bis 30 Gew.-%: einer bei Raumtemperatur flüssigen weiteren Epoxidverbindung oder eines Gemisches mehrerer derartiger Epoxidverbindungen sowie
- 0 bis 25 Gew.-%: einer bei Raumtemperatur festen weiteren Epoxidverbindung oder eines Gemisches mehrerer derartiger Epoxidverbindungen,
wobei sich die Prozentangaben auf die Gesamtmenge von Epoxidverbindungen in der Zusammensetzung beziehen und zu maximal 100 Gewichtsprozent ergänzen.

Die Herstellung der festen Mischphasen kann z.B. erfolgen, indem man ein Gemisch der festen und flüssigen Epoxidharze erhitzt, bis die Mischung vollkommen flüssig ist, die Mischung danach gegebenenfalls homogenisiert, abkühlt und das erhaltene Produkt gegebenenfalls zerkleinert, oder indem man feste und flüssige Epoxidharze gemeinsam in einem inerten Lösungsmittel auflöst und danach das Lösungsmittel entfernt, bis die festen Mischphasen ausfallen, die dann isoliert werden können. Feste Lösungen bestimmter Epoxidharze, insbesondere von Polyglycidylpolycarbonsäureestern lassen sich auch in besonders eleganter Weise erhalten, indem man die Polycarbonsäuren homogen vermischt und das erhaltene Gemisch danach in üblicher Weise glycidylisiert. Die genannten Herstellungsverfahren sind in der schon zitierten EP-A-0 536 085 eingehend beschrieben.

Für die erfindungsgemässen Pulverlacke geeignete Poly(meth)acrylharze mit freien Carboxylgruppen lassen sich in bekannter Weise durch Copolymerisation von Acrylund/oder Methacrylmonomeren, also beispielsweise von C₁-C₁₂-Alkyl(meth)acrylaten, wie Methyl-, Ethyl-, Propyl-, Butyl-, Pentyl-, Hexyl-, Octyl-, 2-Ethylhexyl-, Decyl-, Dodecyl(meth)acrylaten, wobei C₁-C₄-Alkyl(meth)acrylate bevorzugt sind, oder (Meth)acrylamid mit Acrylsäure und/oder Methacrylsäure und gegebenenfalls zusätzlichen anderen ethylenisch ungesättigten Comonomeren, wie Vinylaromaten, z.B. Styrol, α-Methylstyrol, Vinyltoluol oder auch β-halogenierten Styrolen, erhalten. Die Copolymerisation kann hierbei in bekannter Weise erfolgen. So kann man z.B. die Monomeren in geeigneten organischen Lösungsmitteln lösen und in Gegenwart eines geeigneten, in dem Lösungsmittel löslichen Initiators, wie Dicumylperoxid, und eines geeigneten Kettenübertragungsreagenzes, wie Thioglycolsäure, thermisch umsetzen (Lösungspolymerisation) oder das Monomerengemisch zusammen mit einer Lösung des Initiators in einem organischen Lösungsmittel in Wasser suspendieren und polymerisieren oder das Monomerengemisch auch mit Hilfe von Tensiden, z.B. Natriumlaurylsulfat in Wasser emulgieren und in Gegenwart eines wasserlöslichen Polymerisationsinitiators, wie K₂S₂O₈, umsetzen (Emulsionspolymerisation). Danach wird jeweils das fertige Poly(meth)acrylharz aus dem Lösungsmittel oder Wasser in fester Form isoliert. Die Umsetzung kann auch ohne die Verwendung von Lösungsmitteln oder Wasser erfolgen, z.B. gemäss JP-A-Sho 53-140395. Geeignete Poly(meth)acrylharze sind bei Temperaturen im Bereich von Raumtemperatur (15 bis 25°C) fest. Sie weisen im allgemeinen ein Molekulargewicht zwischen 1000 und 50000 (Gewichtsmittel M_{w}) bevorzugt zwischen 5000 und 20000 auf.

Der Tg-Wert (Glasübergangstemperatur) der Poly(meth)acrylharze, bestimmt mit DSC (Aufheizgeschwindigkeit 10°C/Minute), beträgt bevorzugt 40 bis 75°C. Die Säurezahl der Harze, angegeben in mg äquivalente KOH pro g (Meth)acrylatharz, beträgt bevorzugt 30 bis 160, besonders bevorzugt 70 bis 140.

Die erfindungsgemässen Pulverlackzusammensetzungen enthalten die Epoxidharze bevorzugt in einer Menge, dass das Verhältnis Carboxylgruppen der Poly(meth)acrylharze zu Epoxidgruppen zwischen 0,5:1 und 2:1 liegt.

Die erfindungsgemässen Pulverlacke können auch noch weitere in der Lackindustrie übliche Zusatzstoffe enthalten, beispielsweise Lichtschutzmittel, Härtungsbeschleuniger, Farbstoffe, Pigmente, z.B. Titandioxidpigment, Entgasungsmittel, z.B. Benzoin, und/oder Verlaufsmittel. Geeignete Verlaufsmittel sind z.B. Polyvinylacetale, wie Polyvinylbutyral, Polyethylenglycol, Polyvinylpyrrolidon, Glycerin, Acrylmischpolymerisate, wie sie z.B. unter den Bezeichnungen Modaflow® oder Acrylron® erhältlich sind.

Erfindungsgemässe Pulverlacke können durch einfaches Mischen der Bestandteile, z.B. in einer Kugelmühle, hergestellt werden. Eine andere und mehr bevorzugte Möglichkeit besteht darin, dass man die Bestandteile zusammen aufschmilzt, vermischt und homogenisiert, vorzugsweise in einer Extrusionsmaschine, wie einem Buss-Kokneter, die Masse abkühlt und zerkleinert. Hierbei erweist es sich als besonders vorteilhaft, dass die erfindungsgemässen Pulverlacke nach der Extrusion entweder gleich zumindest aber nachdem man sie einige Stunden, beispielsweise 24 bis 48 Stunden, stehen gelassen hat, so hart und spröde werden, dass sie ohne weiteres vermahlen werden können. Die Pulverlackmischungen weisen vorzugweise eine Partikelgrösse im Bereich von 0,015 bis 500 µm, insbesondere von 10 bis 75 µm, auf. In manchen Fällen kann es auch zweckmässig sein, zunächst ein Masterbatch aus Teilen des Bindemittels, der Epoxidharze und gegebenenfalls weiterer Komponenten herzustellen, das dann in einem zweiten Schritt mit dem Rest des Bindemittels und den restlichen Bestandteilen zu endgültigen Pulverlackzusammensetzung abgemischt und homogenisiert wird.

Die Pulverlacke werden nach ihrer Applikation auf den zu beschichtenden Gegenstand bei einer Temperatur von mindestens etwa 100°C, vorzugsweise von 120 bis 140°C, gehärtet. Für die Härtung sind im allgemeinen etwa 5 bis 60 Minuten erforderlich. Zur Beschichtung geeignet sind alle Materialien, die bei den zur Härtung erforderlichen Temperaturen stabil sind, insbesondere Keramik und Metall. Das Substrat kann hierbei bereits eine oder mehrere Basislackbeschichtungen aufweisen, die mit dem Pulverlack verträglich sind.

Ein bevorzugtes Anwendungsgebiet für erfindungsgemässe Zusammensetzungen sind Automobildecklacke, d.h. Klarlacke, die als oberste Schicht auf einer oder mehren Basislackschichten aufgebracht werden, mit denen das Automobil zunächst versehen wird. Ein weiterer Gegenstand der Erfindung ist daher die Verwendung der oben beschriebenen Pulverlackzusammensetzungen als Automobildecklack.

Beispiel 1: In einem Extruder (Laborextruder der Firma PRISM, The Old Stables, England) werden bei einer Temperatur zwischen 60 und 80°C die in der Tabelle 1 angegebenen Pulverlackzusammensetzungen (Mengen in Gramm) homogenisiert. Die abgekühlten Extrudate werden zum fertigen Pulverlack mit einer Partikelgrösse von etwa 40 µm vermahlen. Die Pulverlackzusammensetzungen werden auf ein mit einem Silbermetallicbasislack (VWL 97A BASF) vorbeschichtetem Aluminiumblech elektrostatisch aufgesprüht, wobei die Schichtstärke der Basislackbeschichtung 40 µm und die des Pulverlacks 60 µm beträgt.

**Tabelle 1**

| Pulverlack Nr. | 1 | 2 | 3 |
|---|---|---|---|
| Joncryl SCX 819(1) | 87 | 87 | 83,8 |
| TGIC(2) | 13 | | |
| trans 1,4 CHDA DGE (3) | | 13 | 16,2 |
| Benzoin | 0,3 | 0,3 | 0,3 |
| Modaflow M 2000 (4) | 0,3 | 0,3 | 0,3 |
| Tinuvin 144(5) | 1,4 | 1,4 | 1,4 |
| Tinuvin 900 (6) | 2,6 | 2,6 | 2,6 |
| Extrusionsbedingungen | PRISM 60-80°C/250 rpm | | |
| Gel Zeit 140°C (s) | 250 | 760 | 540 |
| Einbrennbedingungen | 30 min/140°C | | |
| Schichtstärke (µm) | 60 | 60 | 60 |
| Glanz (20°) | 80 | 80 | 87 |
| Glanz (60°) | 100 | 100 | 98 |
| Verlauf (visuell) (7) | 1 | 7 | 7 |
| Verlauf LW (8) | 61,5 | 41,4 | 33 |
| Verlauf SN (9) | 153,7 | 88,8 | 75 |
| Tg °C Pulver (10) | 37 | 30 | 25 |
| Tg °C Gehärtetes Syst. | 46 | 55 | 47 |

| | | | |
|---|---|---|---|
| (1): Carboxylfunktionelles Acrylatharz der Firma SC Johnson Wax; Säurezahl: 75 mg KOH/g Substanz; ICI-Schmelzviskosität: 6000 Mpas bei 200°C; Molekulargewicht (Gewichtsmittel M_{w}): 17000 | | | |
| (2): Triglycidylisocyanurat | | | |
| (3): trans 1,4-Cyclohexandicarbonsäurediclycidylester | | | |
| (4) Verlaufsmittel auf Basis eines butylierten Polyacrylats | | | |
| (5): HALS (Polyalkylpiperidinverbindung) | | | |
| (6): UV-Absorber | | | |
| (7): Empirische Skala von 0 (mangelhaft) bis 10 (sehr gut) Verlaufbestimmung mit Hilfe des "Wave Scan" - Profilometer (Fa. Byk) | | | |
| (8): Parameter "Long wave": von >60 (mangelhaft) bis <30 (sehr gut) | | | |
| (9): Parameter "Subnote": von >150 (mangelhaft) bis <50 (sehr gut) | | | |
| (10): Tg-Werte dieser Tabelle wurden mit DSC (Heizgeschwindigkeit: 10°C/min) bestimmt. | | | |

Man kann der Tabelle 1 insbesondere entnehmen, dass das Verlaufsverhalten der erfindungsgemässen Pulverlacke Nr. 2 und 3 wesentlich besser ist als das eines herkömmlichen Pulverlackes mit Triglycidylisocyanurat als Härter (Pulverlack 1).

### Beispiel 2: Ein Methacrylharz wird in der nachfolgend geschriebenen Weise hergestellt:

| Momomerenbeschickung: | Gewichtsteile [g] |
|---|---|
| - Methacrylsäure (MAA) | 30 |
| - Styrol (St) | 20 |
| - Methylmethacrylat (MMA) | 30 |
| - Butylmethacrylat (BuMA) | 120 |
| -Dicumylperoxid (Initiator) | 3,0 |
| - Thioglycolsäure (Kettenübertragungsmittel) | 3,5 |

| Vorlage: | |
|---|---|
| - Toluol | 60 Milliliter |
| - Dicumylperoxid (Initiator) | 3,0 g |
| - Thioglycolsäure (Kettenübertragungsmittel) | 3,5 g |

Das als Monomerenbeschickung dienende Gemisch wird mit einer 4%-igen Natriumhydroxid- und einer 20%-igen Natriumchloridlösung gewaschen und über wasserfreiem Natriumsulfat getrocknet.

Die Vorlage wird in ein Reaktionsgefäss mit mechanischem Rührer, Stickstoffspülung, Kühler, Heizung, Thermometer und einer Dosierpumpe zur kontinuierlichen Zugabe der Monomerenbeschickung gegeben. Unter Stickstoffspülung wird die Temperatur der Vorlage auf 110°C erhöht, so dass das Toluol unter Rückfluss siedet. Dann wird die Monomerenbeschickung während eines Zeitraums von 3 Stunden zur Vorlage in dem Reaktionsgefäss gegeben, wobei gegen Ende der Zugabe die Viskosität der Mischung im Reaktionsgefäss stark zunimmt. Nach weiterem etwa sechstündigem Erhitzen der Mischung auf 110°C wird auf Raumtemperatur abgekühlt. Das Toluol wird verdampft, das zurückbleibende Copolymer in Ether gelöst, mit Hexan gefällt, abfiltriert und getrocknet.

Das erhaltene Copolymer hat ein Molekulargewicht (M_{w}) von 10330 (gemessen mit GPC unter Verwendung eines Polystyrolstandards; M_{w}/Mₙ = 4,7), eine Glasübergangstemperatur (T_{g}-Wert) von 45,8°C und weist 1,98 Äquivalente freie Carboxylgruppen pro Kilogramm Copolymer auf.

In einem Extruder (Laborextruder der Firma PRISM, The Old Stables, England) werden bei einer Temperatur zwischen 60 und 80°C die in der Tabelle 2 angegebenen Pulverlackzusammensetzungen (Mengen in Gramm) homogenisiert. Die abgekühlten Extrudate werden zum fertigen Pulverlack mit einer Partikelgrösse von etwa 40 µm vermahlen. Die Pulverlackzusammensetzungen werden auf ein mit einem Silbermetallicbasislack (VWL 97A BASF) vorbeschichtetem Aluminiumblech elektrostatisch aufgesprüht, wobei die Schichtstärke der Basislackbeschichtung 40 µm und die des Pulverlacks 60 µm beträgt.

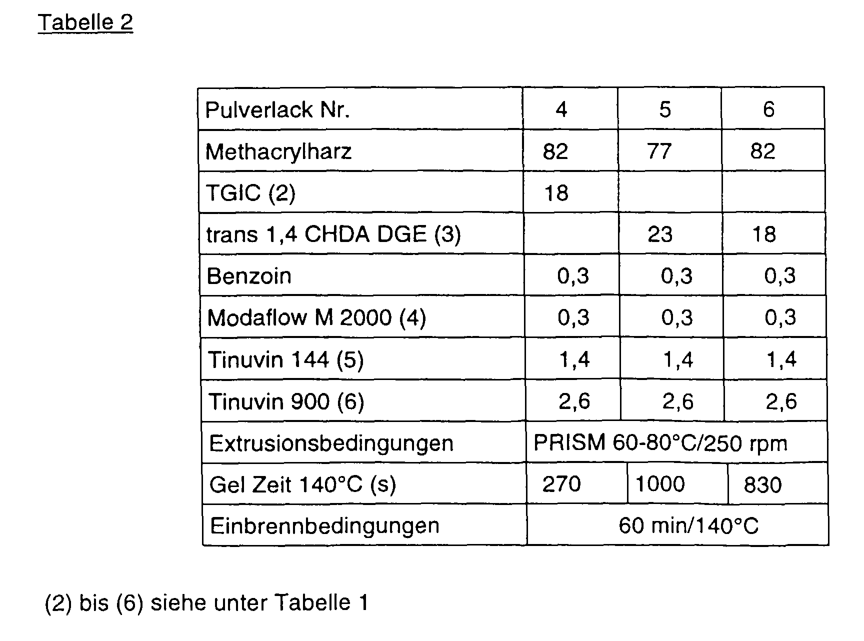

Aus Tabelle 2 (Fortsetzung) kann man wiederum entnehmen, dass das Verlaufsverhalten sowie der Glanz der erfindungsgemässen Pulverlacke Nr. 5 und 6 sehr gut ist. Ein Pulverlack auf Basis des gleichen Methacrylharzes als Bindemittel und mit Triglycidylisocyanurat als Härter (Pulverlack 4) zeigt dagegen bei einem Winkel von 20° einen derart schlechten Glanz, dass er in der Praxis nicht eingesetzt werden könnte.

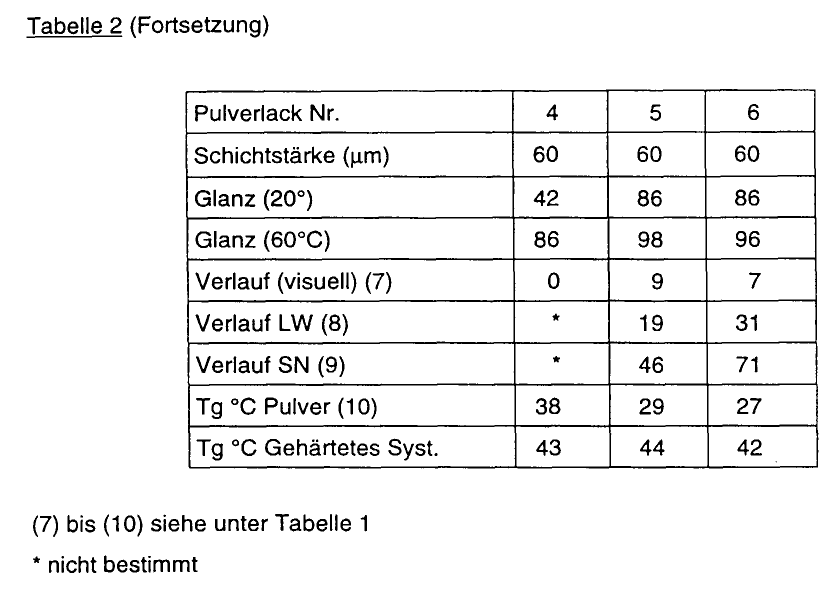

### Beispiel 3: Gemäss Beispiel 2 wird unter Verwendung der nachfolgend beschriebenen Monomerenbeschickung und Vorlage das Methacrylharz A hergestellt:

| Momomerenbeschickung: | Gewichtsteile [g] |
|---|---|
| - Methacrylsäure (MAA) | 15 |
| - Styrol (St) | 15 |
| - Methylmethacrylat (MMA) | 30 |
| - Butylmethacrylat (BuMA) | 40 |
| - Dicumylperoxid (Initiator) | 1,5 |
| - Thioglycolsäure (Kettenübertragungsmittel) | 1,75 |

| Vorlage: | |
|---|---|
| - Toluol | 30 Milliliter |
| - Dicumylperoxid | 1,5 g |
| - Thioglycolsäure | 1,75g |

67 g trans 1,4-Cyclohexandicarbonsäurediclycidylester werden mit 33 g Hexahydro-trimellitsäuretriglycidylester gemischt. Das Gemisch wird erwärmt, bis eine klare Schmelze erhalten wird. Dann wird das Gemisch abgekühlt. Man erhält eine feste Lösung der beiden Glycidylester (Feste Lösung I).

Die in der Tabelle 3 angegebenen Pulverlackzusammensetzungen (Mengen in Gramm) werden gemäss Beispiel 2 homogenisiert und vermahlen. Die Pulverlacke werden auf ein mit einem Silbermetallicbasislack (VWL 97A BASF) vorbeschichtetes Aluminiumblech elektrostatisch aufgesprüht, wobei die Schichtstärke der Basislackbeschichtung 40 µm beträgt und die des Pulverlacks in Tabelle 3 angegeben ist.

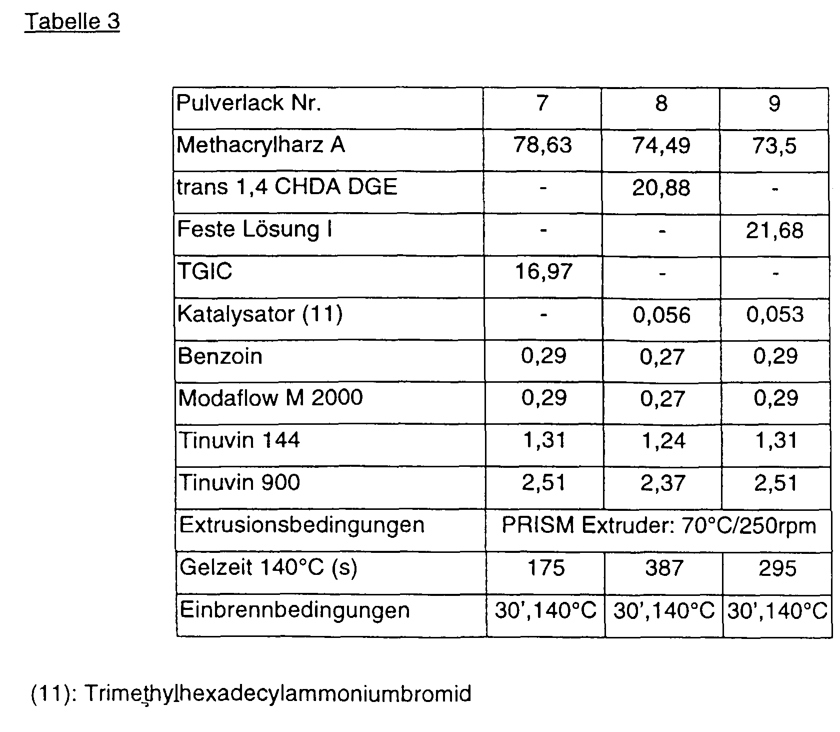

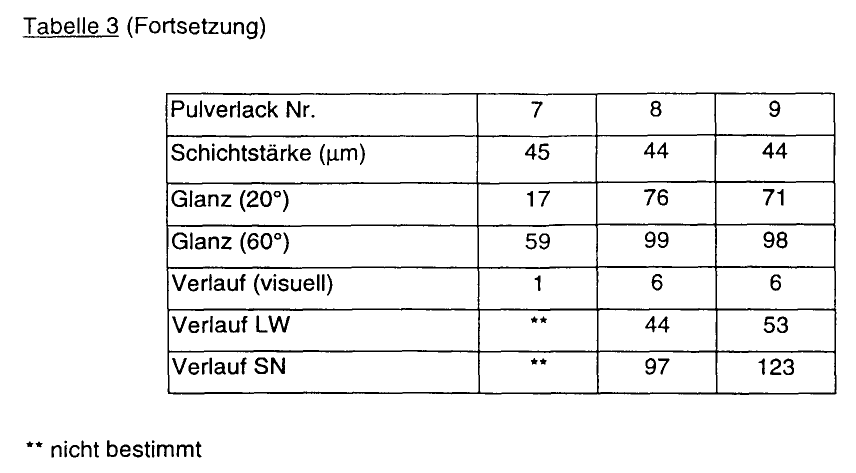

Aus Tabelle 3 (Fortsetzung) kann man wiederum entnehmen, dass das Verlaufsverhalten sowie der Glanz der erfindungsgemässen Pulverlacke Nr. 8 und 9 gut sind. Ein Pulverlack auf Basis des gleichen Methacrylharzes als Bindemittel und mit Triglycidylisocyanurat als Härter (Pulverlack 7) zeigt dagegen bei einem Winkel von 20° einen derart schlechten Glanz, dass er in der Praxis nicht eingesetzt werden könnte.

## Patentansprüche

1. Pulverlack auf Basis eines oder mehrerer Poly(meth)acrylharze mit freien Carboxylgruppen, enthaltend einen Härter auf Basis von Epoxidverbindungen mit einem Molekulargewicht von höchstens 1500, der bei Raumtemperatur fest ist, wobei die Epoxidverbindungen aus 60 bis 100 Gewichtsprozent (Gew.-%) 1,4-Cyclohexandicarbonsäurediglycidylester in Form des reinen trans-lsomeren oder eines cis-/trans-Isomerengemisches mit einem Schmelzpunkt (Peakmaximum im DSC-Scan bei einer Aufheizgeschwindigkeit von 10°C pro Minute) oberhalb von 70°C und 0 bis 40 Gewichtsprozent weiteren Epoxidverbindungen bestehen, wobei 0 bis 15 Gewichtsprozent der gesamten Epoxidverbindungen aromatische Strukturelemente im Molekül aufweisen können.

2. Pulverlack nach Anspruch 1, bei dem die Epoxidverbindungen zu 100 Gewichtsprozent aus 1,4-Cyclohexandicarbonsäurediglycidylester in Form eines reinen trans-Isomeren oder eines cis-/trans cis-/trans-Isomerengemisches mit einem Schmelzpunkt oberhalb von 70°C bestehen.

3. Pulverlack nach Anspruch 1, bei dem die weiteren Epoxidverbindungen sämtlich aus aliphatischen und cycloaliphatischen Epoxidverbindungen ausgewählt sind.

4. Pulverlack nach Anspruch 1 oder 3, bei dem die weiteren Epoxidverbindungen aus Di- und Polyglycidylestern ausgewählt sind.

5. Pulverlack nach Anspruch 1, 3 oder 4, bei dem die weiteren Epoxidverbindungen bei Raumtemperatur fest sind.

6. Pulverlack nach Anspruch 1, 3 oder 4, bei dem die Epoxidverbindungen eine bei Raumtemperatur feste Zusammensetzung enthaltend bei Raumtemperatur feste und bei Raumtemperatur flüssige Epoxidverbindungen bilden, wobei zumindest ein Teil der bei Raumtemperatur festen Epoxidverbindungen in Form einer festen Mischphase oder eines Gemisches mehrerer fester Mischphasen vorliegt und die gesamten bei Raumtemperatur flüssigen Epoxidverbindungen Komponenten einer oder mehrerer der genannten festen Mischphasen bilden.

7. Pulverlack nach Anspruch 6, bei dem die feste Zusammensetzung die Epoxidverbindungen in folgendem Verhältnis enthalten:
70 bis 95 Gew.-% 1,4-Cyclohexandicarbonsäurediglycidylester in Form des reinen trans-Isomeren oder eines cis-/trans cis-/trans-Isomerengemisches mit einem Schmelzpunkt oberhalb von 70°C,
5 bis 30 Gew.-% einer bei Raumtemperatur flüssigen weiteren Epoxidverbindung oder eines Gemisches mehrerer derartiger Epoxidverbindungen sowie
0 bis 25 Gew.-% einer bei Raumtemperatur festen weiteren Epoxidverbindung oder eines Gemisches mehrerer derartiger Epoxidverbindungen,
wobei sich die Prozentangaben auf die Gesamtmenge von Epoxidverbindungen in der Zusammensetzung beziehen und zu maximal 100 Gewichtsprozent ergänzen.

8. Pulverlack nach einem der Ansprüche 1 bis 7, bei dem der Tg-Wert der Poly(meth)acrylharze mit freien Carboxylgruppen, bestimmt mit DSC (Aufheizgeschwindigkeit 10°C pro Minute), 40 bis 75°C beträgt.

9. Pulverlack nach einem der Ansprüche 1 bis 8, bei dem die Säurezahl der Poly(meth)acrylharze mit freien Carboxylgruppen, angegeben in Milligramm äquivalente KOH pro Gramm Poly(meth)acrylharz, 30 bis 160 beträgt.

10. Pulverlack nach Anspruch 9, bei dem die Säurezahl der Poly(meth)acrylharze mit freien Carboxylgruppen, angegeben in Milligramm äquivalente KOH pro Gramm Poly(meth)acrylharz, 70 bis 140 beträgt.

11. Automobildecklack nach einem der Ansprüche 1 bis 10.

## Claims

1. A powder coating material based on one or more poly(meth)acrylic resins having free carboxyl groups, comprising a hardener based on epoxide compounds having a molecular weight of no more than 1500, which is solid at room temperature, the epoxide compounds consisting of 60 to 100% (percent) by weight of diglycidyl 1,4-cyclohexanedicarboxylate in the form of the pure trans-isomer or of a cis/-trans-isomer mixture having a melting point (peak maximum of the DSC scan at a heating rate of 10°C per minute) of above 70°C, and of 0 to 40% by weight of other epoxide compounds, and wherein 0 to 15% by weight of the entire amount of epoxide compounds can contain aromatic structural elements in the molecule.

2. A powder coating material according to claim 1, wherein the epoxide compounds consist 100% by weight of diglycidyl 1,4-cyclohexanedicarboxylate in the form of a pure trans-isomer or of a cis-/trans-isomer mixture having a melting point of above 70°C.

3. A powder coating material according to claim 1, wherein the other epoxide compounds are all selected from aliphatic and cycloaliphatic epoxide compounds.

4. A powder coating material according to claim 1 or 3, wherein the other epoxide compounds are selected from di- and polyglycidyl esters.

5. A powder coating material according to claim 1, 3 or 4, wherein the other epoxide compounds are solid at room temperature.

6. A powder coating material according to claim 1, 3 or 4 wherein the epoxide compounds form a composition which is solid at room temperature, comprising epoxide compounds which are solid at room temperature and epoxide compounds which are liquid at room temperature, and wherein at least part of those epoxide compounds which are solid at room temperature is in the form of a solid mixed phase or of a mixture of two or more solid mixed phases and wherein all those epoxide compounds which are liquid at room temperature are components of one or more of the said solid mixed phases.

7. A powder coating material according to claim 6, wherein the solid composition contains the epoxide compounds in the following proportion:
70-95% by weight of glycidyl 1,4-cyclohexanedicarboxylate in the form of the pure trans-isomer or of a cis-/trans-isomer mixture having a melting point of above 70°C,
5-30% by weight of another epoxide compound which is liquid at room temperature or of a mixture of two or more such epoxide compounds, and
0-25% by weight of another epoxide compound which is solid at room temperature or of a mixture of two or more such epoxide compounds,
the percentages relating to the entire amount of epoxide compounds in the composition and adding up to a maximum of 100% by weight.

8. A powder coating material according to one of claims 1 to 7, wherein the Tg value of the poly(meth)acrylic resins having free carboxyl groups, determined by DSC (heating rate 10°C per minutes), is from 40 to 75°C.

9. A powder coating material according to one of claims 1 to 8, wherein the acid number of the poly(meth)acrylic resins having free carboxyl groups, stated in milligram equivalents of KOH per gram of poly(meth)acrylic resin, is from 30 to 160.

10. A powder coating material according to claim 9, wherein the acid number of the poly(meth)acrylic resins having free carboxyl groups, stated in milligramm equivalents of KOH per gram of poly(meth)acrylic resin, is from 70 to 140.

11. An automotive topcoat material according to one of claims 1 to 10.

## Revendications

1. Peinture en poudre à base d'une ou plusieurs résines poly(méth)acryliques ayant des groupes carboxyle libres, contenant un durcisseur à base de composés époxy ayant une masse molaire inférieure ou égale à 1 500, qui est solide à la température ambiante, les composés époxy étant constitués de 60 à 100 % en masse de 1,4-cyclohexanedicarboxylate de diglycidyle sous forme de l'isomère trans pur ou d'un mélange d'isomères cis/trans ayant un point de fusion (défini comme la valeur maximale en calorimétrie différentielle à balayage (CDB) à une vitesse de chauffage de 10°C par minute) supérieur à 70°C, et de 0 à 40 % en masse d'autres composés époxy, 0 à 15 % en masse de l'ensemble des composés époxy pouvant contenir des éléments de structure aromatiques dans la molécule.

2. Peinture en poudre selon la revendication 1, dans laquelle les composés époxy sont constitués à 100 % en masse de 1,4-cyclohexanedicarboxylate de diglycidyle sous forme de l'isomère trans pur ou d'un mélange d'isomères cis/trans ayant un point de fusion supérieur à 70°C.

3. Peinture en poudre selon la revendication 1, dans laquelle les autres composés époxy sont tous choisis parmi des composés époxy aliphatiques et cycloaliphatiques.

4. Peinture en poudre selon la revendication 1 ou la revendication 3, dans laquelle les autres composés époxy sont choisis parmi des esters di- et polyglycidyliques.

5. Peinture en poudre selon la revendication 1, 3 ou 4, dans laquelle les autres composés époxy sont solides à la température ambiante.

6. Peinture en poudre selon la revendication 1, 3 ou 4, dans laquelle les composés époxy forment une composition solide à la température ambiante contenant des composés époxy solides à la température ambiante et des composés époxy liquides à la température ambiante, au moins une partie des composés époxy solides à la température ambiante se trouvant sous forme d'une phase mixte solide ou d'un mélange de plusieurs phases mixtes solides et l'ensemble des composés époxy liquides à la température ambiante formant des constituants d'une ou plusieurs desdites phases mixtes solides.

7. Peinture en poudre selon la revendication 6, dans laquelle la composition solide contient les composés époxy dans les proportions suivantes:
70 à 95 % en masse de 1,4-cyclohexanedicarboxylate de diglycidyle sous forme de l'isomère trans pur ou d'un mélange d'isomères cis/trans ayant un point de fusion supérieur à 70°C,
5 à 30 % en masse d'un autre composé époxy liquide à la température ambiante ou d'un mélange de plusieurs composés époxy de ce type, et
0 à 25 % en masse d'un autre composé époxy solide à la température ambiante ou d'un mélange de plusieurs composés époxy de ce type,
les indications en pourcentage se rapportant à la quantité totale de composés époxy dans la composition et totalisant au maximum 100 % en masse.

8. Peinture en poudre selon l'une des revendications 1 à 7, dans laquelle la valeur de Tg des résines poly(méth)acryliques à groupes carboxyle libres, déterminée par CDB (vitesse de chauffage de 10°C par minute) est comprise entre 40 et 75°C.

9. Peinture en poudre selon l'une des revendications 1 à 8, dans laquelle l'indice d'acide des résines poly(méth)acryliques à groupes carboxyle libres, indiqué en mg de KOH équivalent par g de résine poly(méth)acrylique, est compris entre 30 et 160.

10. Peinture en poudre selon la revendication 9, dans laquelle l'indice d'acide des résines poly(méth)acryliques à groupes carboxyle libres, indiqué en mg de KOH équivalent par g de résine poly(méth)acrylique, est compris entre 70 et 140.

11. Peinture de finition pour automobiles selon l'une des revendications 1 à 10.
